Europäisches Patentamt

European Patent Office

Office européen des brevets

(11) Veröffentlichungsnummer: **0 292 674 B1**

(12) **EUROPÄISCHE PATENTSCHRIFT**

(45) Veröffentlichungstag der Patentschrift: **11.11.92**

(51) Int. Cl.⁵: **C07C 33/042**, C07C 29/00

(21) Anmeldenummer: **88105336.7**

(22) Anmeldetag: **02.04.88**

(54) **Verfahren zur Herstellung von Propinol.**

(30) Priorität: **23.05.87 DE 3717471**
**23.05.87 DE 3717470**
**23.05.87 DE 3717468**

(43) Veröffentlichungstag der Anmeldung:
**30.11.88 Patentblatt 88/48**

(45) Bekanntmachung des Hinweises auf die
Patenterteilung:
**11.11.92 Patentblatt 92/46**

(84) Benannte Vertragsstaaten:
**AT BE CH DE FR GB IT LI LU NL SE**

(56) Entgegenhaltungen:
**DE-B- 1 174 765**
**US-A- 3 636 167**

(73) Patentinhaber: **GAF-HüLS CHEMIE GMBH**
**Postfach 13 20**
**W-4370 Marl(DE)**

(72) Erfinder: **Westernacher, Helmut, Dr.**
**Burgstrasse 12**
**W-4358 Haltern(DE)**
Erfinder: **Aertken, Karl**
**Heidelohstrasse 13**
**W-4408 Dülman(DE)**
Erfinder: **Stieren, Thomas**
**Marler Strasse 102**
**W-4358 Haltern(DE)**

EP 0 292 674 B1

## Beschreibung

Die Erfindung betrifft ein Verfahren zur Herstellung von Propinol durch katalytische Spaltung von Butindiol-(1,4).

Propinol wird zur Synthese von Polyinen und von anderen Naturstoffen eingesetzt und dient außerdem als Korrosionsschutzmittel in der Galvanotechnik.

Bei der Herstellung von Butindiol-(1,4) aus Formaldehyd und Acetylen fällt Propinol in kleinen Mengen als Nebenprodukt an.

Bei den bekannten Verfahren zur Herstellung von Propinol geht man ebenfalls von Formaldehyd und Acetylen aus. Die Folgereaktion von Propinol mit Formaldehyd zu Butindiol-(1,4) wird dabei durch spezielle Reaktionsbedingungen unterdrückt.

In DE-PS 11 74 765 wird ein Zusatz von N-Methyl-pyrrolidon geschützt, das ein gutes Lösemittel für Acetylen ist und deshalb eine hohe Acetylenkonzentration ermöglicht. Die Reaktion von Acetylen und Formaldehyd in Gegenwart von Kupferacetylid führt hier überwiegend zu Propinol. Es werden dabei jedoch auch erhebliche Mengen an Butindiol-(1,4) erhalten. Der Aufwand zur Isolierung von reinem Propinol aus dem Reaktionsgemisch ist erheblich.

Nach DE-PS 12 84 964 können Acetylen und Formaldehyd in einem Lösemittel, beispielsweise in Butyrolacton, mit Hilfe von Kupferacetylid auf einem Träger zu Butindiol-(1,4) und Propinol umgesetzt werden. Dabei werden beide Produkte in vergleichbaren Mengen gebildet. Bei dieser Reaktion werden hohe Acetylendrücke angewendet, die vor allem bei großtechnischen Anlagen einen beträchtlichen sicherheitstechnischen Aufwand erfordern.

Aufgabe der vorliegenden Erfindung ist es, ein Verfahren bereitzustellen, durch das Propinol unter vermindertem technischen Aufwand in verbesserter Ausbeute und Reinheit hergestellt werden kann.

Die Aufgabe wird erfindungsgemäß dadurch gelöst, daß man Butindiol-(1,4) bei erhöhter Temperatur mit Hilfe eines Kupferacetylidkatalysators zu Propinol und Formaldehyd spaltet und Propinol aus der Reaktionsmischung entfernt.

Eine bevorzugte Ausführungsform besteht darin, daß man Butindiol-(1,4) bei erhöhter Temperatur in einem hochsiedenden organischen Lösemittel mit Hilfe eines Kupferacetylidkatalysators zu Propinol und Formaldehyd spaltet und Propinol destillativ abtrennt.

Für die Reaktion nach dieser bevorzugten Ausführungsform kann wasserfreies Butindiol-(1,4) eingesetzt werden. Ist das Ausgangsprodukt wasserhaltig, so muß es vor der Reaktion entwässert werden, was beispielsweise durch Erhitzen bei vermindertem Druck erfolgen kann.

Der für diese Reaktion günstige Temperaturbereich liegt bei 130 bis 180 °C. Vorzugsweise wird die Reaktion bei 140 bis 170 °C durchgeführt. Bei zu niedriger Temperatur ist die Reaktionsgeschwindigkeit für ein technisches Verfahren zu gering. Bei zu hoher Temperatur treten verstärkt Nebenreaktionen und Verharzungen auf, so daß die Ausbeute an Propinol vermindert wird.

Für die Reaktion geeignete Lösemittel haben unter Normalbedingungen Siedepunkte im Bereich von etwa 130 bis 240 °C. Es sind beispielsweise Ethylenglykol, Hexanol, Octanol und N-Methylpyrrolidon. Vorzugsweise wird Ethylenglykol verwendet.

Wird die Reaktion bei unter Rückfluß siedendem Lösemittel durchgeführt, so wird dadurch die destillative Abtrennung des gebildeten Propinols erleichtert. Die Reaktion kann bei Normaldruck durchgeführt werden. Zur Einstellung eines bestimmten Siedepunktes kann auch ein leichtes Vakuum bis etwa 300 mbar angelgt werden.

Die eingesetzte Lösung enthält 20 bis 80 Gew.-% Butindiol-(1,4). Vorzugsweise liegt die Konzentration bei 25 bis 50 Gew.-%. Bei Konzentrationen von unter 20 % ist der Lösemittelgehalt unwirtschaftlich hoch. Bei Konzentrationen von über 80 % werden die Lösungen hochviskos und dadurch schlecht handhabbar. Die Verharzungsgefahr ist erhöht.

In dieser Ausführungsform kann das erfindungsgemäße Verfahren kontinuierlich und diskontinuierlich durchgeführt werden. So kann die Reaktion in einer Apparatur aus Reaktionskolben und aufgesetzter Kolonne bei unter Rückfluß siedendem Lösemittel ausgeführt werden. Dabei reichert sich Propinol am Kopf der Kolonne an und kann dort kontinuierlich abgezogen werden. Gleichzeitig können die Verluste an Ausgangsprodukt durch Zutropfen einer Schmelze oder einer wäßrigen Lösung an Butindiol-(1,4) kontinuierlich ersetzt werden. Die Reinheit des erhaltenen Propinols ist dabei abhängig vom gewählten Rücklaufverhältnis und von der Trennfähigkeit der Säule.

Eine weitere, bevorzugte Ausführungsform besteht darin, daß man Butindiol-(1,4) bei erhöhter Temperatur in Gegenwart eines Kupferacetylidkatalysators zu Propinol und Formaldehyd spaltet und Propinol mit Hilfe eines Treibmittels bei einem Druck von 10 bis 200 mbar aus der Reaktionsmischung entfernt.

Für die Reaktion nach dieser bevorzugten Ausführungsform wird im allgemeinen wasserfreies Butindiol-(1,4) eingesetzt. Soll ein wasserhaltiges Treibmittel verwendet werden, kann auch wasserhaltiges Butindiol-(1,4) eingesetzt werden.

Der für diese Reaktion günstige Temperaturbereich liegt bei 120 bis 170 °C. Vorzugsweise wird die Reaktion bei 130 bis 155 °C durchgeführt. Bei zu niedriger Temperatur ist die Reaktionsgeschwindigkeit für ein technisches Verfahren zu gering. Bei zu hoher Temperatur treten verstärkt Nebenreaktionen und Verharzungen auf, so daß die Ausbeute an Propinol vermindert wird.

Man entfernt Propinol aus der Reaktionsmischung mit Hilfe eines in die Flüssigkeit eingeleiteten Treibmittels bei vermindertem Druck. Geeignete Treibmittel sind beispielsweise Pentanol, Butanol, Propanol, Ethanol und Wasser. Vorzugsweise wird n-Butanol oder Wasser als Treibmittel verwendet.

Der Druck beträgt im allgemeinen 10 bis 200 mbar, vorzugsweise 30 bis 100 mbar.

Das ausgetriebene Propinol-Treibmittel-Gemisch wird durch Kühlung kondensiert, worauf Propinol durch fraktionierte Destillation oder durch andere übliche Methoden vom Treibmittel abgetrennt wird.

In dieser Ausführungsform kann das Verfahren kontinuierlich oder diskontinuierlich durchgeführt werden. In der Reaktionsmischung können die Verluste, die durch den Umsatz von Butindiol-(1,4) entstehen, durch Zugabe von Butindiol-(1,4) zusammen mit dem Treibmittel wieder ausgeglichen werden.

Eine weitere, bevorzugte Ausführungsform besteht darin, daß man Butindiol-(1,4) bei erhöhter Temperatur und Überdruck in einem niedrigsiedenden organischen Lösemittel mit Hilfe eines Kupferacetylidkatalysators in Gegenwart von Acetylen zu Propinol und Formaldehyd spaltet und Propinol destillativ abtrennt.

Für die Reaktion nach dieser bevorzugten Ausführungsform kann wasserfreies Butindiol-(1,4) eingesetzt werden. Ist das Ausgangsprodukt wasserhaltig, so muß es vor der Reaktion entwässert werden, was beispielsweise durch Erhitzen bei vermindertem Druck erfolgen kann.

Der für diese Reaktion günstige Temperaturbereich liegt bei 130 bis 170 °C. Vorzugsweise wird die Reaktion bei 140 bis 160 °C durchgeführt. Bei zu niedriger Temperatur ist die Reaktionsgeschwindigkeit für ein technisches Verfahren zu gering. Bei zu hoher Temperatur treten verstärkt Nebenreaktionen und Verharzungen auf, so daß die Ausbeute an Propinol vermindert wird.

Für die Reaktion geeignete Lösemittel haben unter Normalbedingungen Siedepunkte im Bereich von etwa 80 bis 129 °C. Es sind beispielsweise n-Butanol, Isobutanol, Propanol und Pentanol. Vorzugsweise wird n-Butanol verwendet.

Wird die Reaktion bei unter Rückfluß siedendem Lösemittel durchgeführt, so wird dadurch die destillative Abtrennung des gebildeten Propinols erleichtert. Dabei erhält man im allgemeinen zunächst ein Propinol-Lösemittel-Gemisch, aus dem Propinol durch fraktionierte Destillation gewonnen werden kann.

Bei der Reaktion wird ein Gesamtdruck von 1,05 bis 5 bar, vorzugsweise von 1,05 bis 2 bar, eingestellt. Dabei wird Acetylen in die Reaktionslösung eingeleitet. Vorzugsweise werden 1 bis 100 l Acetylen pro Stunde und Liter Lösung eingeleitet.

Die eingesetzte Lösung enthält 20 bis 90 Gew.-% Butindiol-(1,4). Vorzugsweise liegt die Konzentration bei 50 bis 70 Gew.-%. Bei Konzentrationen von unter 20 % ist der Lösemittelgehalt unwirtschaftlich hoch. Bei Konzentrationen von über 90 % werden die Lösungen hochviskos und dadurch schlecht handhabbar. Die Verharzungsgefahr ist erhöht.

In dieser Ausführungsform kann das erfindungsgemäße Verfahren kontinuierlich und diskontinuierlich durchgeführt werden. So kann die Reaktion in einer Apparatur aus Reaktionskolben und aufgesetzter Kolonne bei unter Rückfluß siedendem Lösemittel ausgeführt werden. Dabei kann angereichertes Propinol oder ein Gemisch aus Propinol und Lösemittel am Kopf der Kolonne kontinuierlich abgezogen werden. Gleichzeitig können die Verluste an Ausgangsprodukten durch Zutropfen von Butindiol-(1,4) und von Lösemittel kontinuierlich ersetzt werden. Die Reinheit des erhaltenen Propinols ist dabei abhängig von der Siedepunktdifferenz, vom gewählten Rücklaufverhältnis und von der Trennfähigkeit der Säule.

Der für das erfindungsgemäße Verfahren geeignete Kupferacetylidkatalysator enthält Kupferacetylid und einen Träger, wie beispielsweise Magnesiumsilikat, und kann unter anderem auch Wismutoxid enthalten. Der Katalysator enthält allgemein 10 bis 50 Gew.-% Kupfer, vorzugsweise 20 bis 35 Gew.-% Kupfer. Die Herstellung von derartigen Katalysatoren wird in DE-PS 27 19 745 beschrieben.

Der Kupferacetylidkatalysator wird in einer Menge von 2 bis 20 Gew.-%, bezogen auf die Reaktionslösung, eingesetzt. Vorzugsweise liegt der Anteil des Kupferacetylidkatalysators bei 5 bis 15 Gew.-%.

Bei unter 2 % Katalysator ist die Reaktionsgeschwindigkeit zu langsam. Bei über 20 % Katalysator wird die Reaktionsgeschwindigkeit nur noch unwesentlich gesteigert, so daß höhere Katalysatorkonzentrationen unwirtschaftlich sind.

Das vorliegende Verfahren hat den Vorteil, daß es in einer einfachen Apparatur durchgeführt werden kann und daß es Propinol bei hohen Umsätzen in hoher Ausbeute und Reinheit liefert.

Beispiel 1

Der benötigte Kupferacetylidkatalysator wird nach dem in DE-PS 27 19 745 beschriebenen Verfahren hergestellt und vor dem Einsatz noch mit Wasser gewaschen und getrocknet. Er weist 35 %

Cu, 3 % Bi$_2$O$_3$ und 43 % Magnesiumsilikat auf.

In einem 1 l Glaskolben mit Rührer, Tropftrichter und Füllkörperkolonne, die etwa 10 theoretische Böden aufweist, werden 500 g Ethylenglykol, 130 g Butindiol-(1,4) und 45 g Kupferacetylidkatalysator unter Rühren und Druckminderung auf etwa 460 mbar bei 162 °C zum Sieden gebracht. Dabei bildet sich Propinol, das sich am Kopf der Kolonne anreichert und dort kontinuierlich bei einem Rücklaufverhältnis von 5 : 1 abgezogen wird. In 4 Stunden werden 55 g Butindiol-(1,4) umgesetzt und durch Zutropfen einer Butindiol-(1,4)-Schmelze ersetzt.

Ausbeute:
34 g Propinol (= 94% der Theorie)
Reinheit nach GC:
99%

Beispiel 2

Es wird der gleiche Kupferacetylidkatalysator wie in Beispiel 1 verwendet.

In ein 2 l Reaktionsgefäß mit Tropftrichter und Rührer werden 1 957 g Butindiol-(1,4) und 60 g Katalysator gegeben. Zunächst wird restliches Wasser in 1 Stunde bei 40 bis 50 °C und etwa 25 mbar entfernt. Danach liegt der Restwassergehalt unter 0,1 %.

Nun wird unter Rühren bei etwa 25 mbar auf 150 °C erhitzt, wobei die Bildung von Propinol beginnt. Dann werden 150 ml/h n-Butanol als Treibmittel aus dem Tropftrichter in die Flüssigphase eingetragen. Das Abgas aus dem Reaktionsgefäß wird in einer Vorlage bei -10 °C kondensiert.

Bei einer Versuchsdauer von 66 Stunden werden 1 590 g Butindiol-(1,4) umgesetzt und 776 g Propinol gebildet. Die Ausbeute an Propinol beträgt danach 75 % der Theorie.

Beispiel 3

Es wird der gleiche Kupferacetylidkatalysator wie in Beispiel 1 verwendet.

In einer 2,1 l Destillationsblase mit Magnetrührer, Einleitrohr und Füllkörperkolonne, die etwa 7 theoretische Böden aufweist, werden in 1 000 g Butindiol-(1,4) und 500 g n-Butanol 30 g Kupferacetylidkatalysator suspendiert. In die Flüssigkeit werden nun 10 l/h Acetylen eingeleitet, worauf auf 140 bis 142 °C zum Sieden erhitzt wird. Am Kolonnenkopf wird ein Druck von 1,08 bis 1,09 bar gemessen.

Bei einem Rücklaufverhältnis von 10 : 1 werden am Kolonnenkopf 70 bis 80 ml/h Destillat aus n-Butanol und Propinol abgezogen. Die Verluste durch Destillation werden dem System durch Zugabe von Butindiol-(1,4) und n-Butanol in den Sumpf der Kolonne wieder ersetzt.

Nach 25 Stunden sind 170 g Butindiol-(1,4) umgesetzt und 94 g Propinol produziert worden. Das sind 85 % der Theorie.

Reines Propinol erhält man aus der Lösung durch fraktionierte Destillation.

**Patentansprüche**

1. Verfahren zur Herstellung von Propinol,
dadurch gekennzeichnet,
daß man Butindiol-(1,4) bei erhöhter Temperatur mit Hilfe eines Kupferacetylidkatalysators zu Propinol und Formaldehyd spaltet und Propinol aus der Reaktionsmischung entfernt.

2. Verfahren nach Anspruch 1,
dadurch gekennzeichnet,
daß man Butindiol-(1,4) bei erhöhter Temperatur in einem hochsiedenden organischen Lösemittel mit Hilfe eines Kupferacetylidkatalysators zu Propinol und Formaldehyd spaltet und Propinol destillativ abtrennt.

3. Verfahren nach Anspruch 2,
dadurch gekennzeichnet,
daß die Reaktion bei 130 bis 180 °C durchgeführt wird.

4. Verfahren nach Anspruch 2,
dadurch gekennzeichnet,
daß man die Reaktion in Lösemitteln mit Siedepunkten im Bereich von 130 bis 240 °C durchführt.

5. Verfahren nach Anspruch 1,
dadurch gekennzeichnet,
daß man lösemittelfreies Butindiol-(1,4) bei erhöhter Temperatur in Gegenwart eines Kupferacetylidkatalysators zu Propinol und Formaldehyd spaltet und Propinol mit Hilfe eines Treibmittels bei einem Druck von 10 bis 200 mbar aus der Reaktionsmischung entfernt.

6. Verfahren nach Anspruch 5,
dadurch gekennzeichnet,
daß Propinol mit Hilfe von n-Butanol oder Wasser bei einem Druck von 30 bis 100 mbar aus der Reaktionsmischung entfernt wird.

7. Verfahren nach Anspruch 1,
dadurch gekennzeichnet,
daß man Butindiol-(1,4) bei erhöhter Temperatur und uberdruck in einem niedrigsiedenden organischen Lösemittel mit Hilfe eines Kupferacetylidkatalysators in Gegenwart von Acetylen zu Propinol und Formaldehyd spaltet und Propinol destillativ abtrennt.

**8.** Verfahren nach Anspruch 7,
dadurch gekennzeichnet,
daß man die Reaktion in Lösemitteln mit Siedepunkten im Bereich von 80 bis 129 °C durchführt.

**9.** Verfahren nach Anspruch 7,
dadurch gekennzeichnet,
daß man während der Reaktion Acetylen in die Lösung einleitet und einen Gesamtdruck von 1,05 bis 5 bar einstellt.

**10.** Verfahren nach Anspruch 1,
dadurch gekennzeichnet,
daß man die Reaktion in Gegenwart eines Kupferacetylidkatalysators, der 10 bis 50 Gew.-% Kupfer enthält, durchführt.

## Claims

**1.** A process for the manufacture of propinol, characterised in that butindiol-1,4 is cleaved at elevated temperature with the aid of a copper acetylide catalyst to form propinol and formaldehyde, and propinol is removed from the reaction mixture.

**2.** A process according to claim 1, characterised in that butindiol-1,4 is cleaved at elevated temperature in a high-boiling organic solvent with the aid of a copper acetylide catalyst to form propinol and formaldehyde, and propinol is separated by distillation.

**3.** A process according to claim 2, characterised in that the reaction is carried out at a temperature of 130 to 180°C.

**4.** A process according to claim 2, characterised in that the reaction is carried out in a solvent having a boiling point in the range from 130 to 240°C.

**5.** A process according to claim 1, characterised in that solvent-free butindiol is cleaved at elevated temperature in the presence of a copper acetylide catalyst to form propinol and formaldehyde, and propinol is removed from the reaction mixture with the aid of a propellent at a pressure of 10 to 200 mbar.

**6.** A process according to claim 5, characterised in that propinol is removed from the reaction mixture with the aid of n-butanol or water at a pressure of 30 to 100 mbar.

**7.** A process according to claim 1, characterised in that butindiol is cleaved at elevated temperature and elevated pressure in a low-boiling organic solvent with the aid of a copper acetylide catalyst in the presence of acetylene to form propinol and formaldehyde, and propinol is separated by distillation.

**8.** A process according to claim 7, characterised in that the reaction is carried out in a solvent having a boiling point in the range from 80 to 129°C.

**9.** A process according to claim 7, characterised in that acetylene is introduced to the solution during the reaction and a total pressure of 1.05 to 5 bar is maintained.

**10.** A process according to claim 1, characterised in that the reaction is carried out in the presence of a copper acetylide catalyst which contains 10 to 50% by weight copper.

## Revendications

**1.** Procédé de préparation de propinol,
caractérisé par le fait qu'on scinde en propinol et en formaldéhyde du butyne-diol-(1,4), à température élevée, à l'aide d'un catalyseur à l'acétylure de cuivre et qu'on élimine le propinol du mélange réactionnel.

**2.** Procédé selon la revendication 1,
caractérisé par le fait qu'on scinde en propinol et en formaldéhyde du butyne-diol-(1,4), à température élevée, dans un solvant organique à point d'ébullition élevé à l'aide d'un catalyseur à l'acétylure de cuivre et qu'on sépare le propinol par distillation.

**3.** Procédé selon la revendication 2,
caractérisé par le fait que l'on effectue la réaction à des températures de 130 à 180°C.

**4.** Procédé selon la revendication 2,
caractérisé par le fait que l'on effectue la réaction dans des solvants présentant des points d'ébullition compris dans un domaine de 130 à 240°C.

**5.** Procédé selon la revendication 1,
caractérisé par le fait que l'on scinde en propinol et en formaldéhyde du butyne-diol-(1,4) exempt de solvant, à température élevée, en présence d'un catalyseur à l'acétylure de cuivre, et que l'on élimine le propinol du mélange réactionnel à l'aide d'un agent d'entraînement sous une pression de 10 à 200 m.bars.

**6.** Procédé selon la revendication 5,

caractérisé par le fait que l'on élimine le propinol du mélange réactionnel à l'aide du n-butanol ou d'eau sous une pression de 30 à 100 m.bars.

7. Procédé selon la revendication 1, caractérisé par le fait qu'en présence d'acétylène, on scinde en propinol et en formaldéhyde du butyne-diol-(1,4) à température élevée et sous une surpression, dans un solvant organique à bas point d'ébullition, à l'aide d'un catalyseur à l'acétylure de cuivre et que l'on sépare le propinol par distillation.

8. Procédé selon la revendication 7, caractérisé par le fait que l'on effectue la réaction dans des solvants présentant des points d'ébullition compris dans un domaine de 80 à 129° C.

9. Procédé selon la revendication 7, caractérisé par le fait que pendant la réaction on fait passer de l'acétylène dans la solution et établit une pression globale de 1,05 à 5 bars.

10. Procédé selon la revendication 1, caractérisé par le fait que l'on effectue la réaction en présence d'un catalyseur à l'acétylure de cuivre qui contient de 10 à 50 % en poids de cuivre.